# EUROPEAN PATENT APPLICATION

(11) **EP 4 814 104 A1**
(43) Date of publication of application: **30.09.2026**
(21) Application number: 24894564.4
(22) Date of filing: 19.11.2024
(51) Int. Cl.: C12N 1/20, A23L 33/135, A23K 10/16, A61K 35/747, A61P 19/02, A61P 29/00, C12R 1/25

(54) **COMPOSITION FOR PREVENTING, AMELIORATING OR TREATING INFLAMMATORY DISEASES COMPRISING LACTOBACILLUS PLANTARUM WIKIM0175**

(30) Priority: 20.11.2023 KR 20230160816
(71) Applicant: Korea Food Research Institute, Wanju-gun, Jeollabuk-do 55365 (KR)
(72) Inventor: CHOI, Hak Jong, Gwangju 62065 (KR); KWON, Min Sung, Gwangju 61052 (KR); OH, Young-joon, Gwangju 62365 (KR); HONG, Sung Wook, Cheongju-si Chungcheongbuk-do 28165 (KR); KIM, Tae Woon, Gwangju 61706 (KR)
(74) Representative: Mewburn Ellis LLP
(86) International application number: PCT/KR2024/018255
(87) International publication number: WO 2025/110672

(57) **Abstract**

The present invention relates to a *Lactobacillus plantarum WiKim0175* strain isolated from Kimchi and a composition comprising same. Lactobacillus plantarum WiKim0175 according to the present invention inhibits inflammatory responses, and thus can have various applications for the prevention, amelioration and treatment of inflammatory diseases.

## Description

### [Technical Field]

The present invention relates to a *Lactobacillus plantarum* strain, which is a lactic acid bacterium isolated from kimchi, and a composition containing the same for preventing, ameliorating, or treating inflammatory diseases.

### [Background Art]

Osteoarthritis is a degenerative disease in which the cartilage tissue protecting the joints is gradually and irreversibly worn away. Approximately 10-15% of the global population and 31% of the adult population in Korea exhibit symptoms of osteoarthritis, and the number of patients worldwide continues to increase due to population aging and increased life expectancy.

Although extensive research and efforts have recently been directed toward controlling osteoarthritis and elucidating the pathological causes thereof, no fundamental treatment method has yet been developed.

To date, except for artificial joint replacement surgery, pain relief through analgesic and anti-inflammatory agents (such as nonsteroidal anti-inflammatory drugs, COX-2 selective inhibitors, combination formulations, and natural product preparations) has accounted for the largest proportion of osteoarthritis treatment methods. Recently, chondroprotective agents (such as hyaluronic acid, glucosamine, and chondroitin) have been developed and are effective in cell protection, shock absorption, cartilage nourishment, and reinforcement of cartilage metabolism. However, the effects thereof remain insufficient in preventing cellular and tissue degeneration, promoting cartilage formation, and inducing regeneration.

In addition, advanced therapeutic approaches such as cell- and tissue-engineered therapies or gene/cell therapies have recently been developed for the treatment of osteoarthritis, but many issues still remain to be resolved before commercialization.

Although various therapies have been introduced due to the continuously increasing demand for osteoarthritis treatment, they still fail to sufficiently satisfy market demand because of limitations in therapeutic efficacy.

In particular, in the case of domestic osteoarthritis therapies in Korea, market share has mainly increased through natural product-based analgesic and anti-inflammatory products. However, for future entry into the global market, there is a need to develop novel amelioration methods using food-derived materials capable of inducing symptom amelioration or preventing osteoarthritis at an early stage.

### [Disclosure]

### [Technical Problem]

An object of the present invention is to provide a novel *Lactobacillus plantarum* WiKim0175 strain deposited under Accession No. KCCM13151P, which exhibits excellent efficacy in preventing or ameliorating inflammatory diseases.

### [Technical Solution]

Accordingly, the present inventors have made efforts to identify a lactic acid bacterial strain exhibiting an effect of preventing or ameliorating inflammatory diseases from traditional fermented foods, and as a result, isolated and identified a novel lactic acid bacterial strain belonging to the genus *Lactobacillus*, namely *Lactobacillus plantarum* WiKim0175, thereby culminating in the present invention.

The present invention provides *Lactobacillus plantarum* WiKim0175.

The present invention provides a food composition for reducing inflammation or improving joint and cartilage health, including, as an active ingredient, a *Lactobacillus plantarum* WiKim0175 strain, a culture thereof, a lysate thereof, or an extract thereof.

The present invention also provides a functional health food composition for reducing inflammation or improving joint and cartilage health, including, as an active ingredient, a *Lactobacillus plantarum* WiKim0175 strain, a culture thereof, a lysate thereof, or an extract thereof.

In order to accomplish the above object, the present invention provides a method of reducing inflammation or improving joint and cartilage health, including administering a composition including a *Lactobacillus plantarum* WiKim0175 strain, a culture thereof, a lysate thereof, or an extract thereof as an active ingredient to a subject.

In addition, the present invention provides the use of a composition including, as an active ingredient, a *Lactobacillus plantarum* WiKim0175 strain, a culture thereof, a lysate thereof, or an extract thereof for the manufacture of a food or functional health food for reducing inflammation or improving joint and cartilage health.

In addition, the present invention provides a lactic acid bacteria starter culture for fermentation, including a *Lactobacillus plantarum* WiKim0175 strain or a culture thereof.

In addition, the present invention provides a feed or feed additive composition including, as an active ingredient, a *Lactobacillus plantarum* WiKim0175 strain, a culture thereof, a lysate thereof, or an extract thereof.

In addition, the present invention provides a pharmaceutical composition for preventing or treating inflammatory diseases, including, as an active ingredient, a *Lactobacillus plantarum* WiKim0175 strain, a culture thereof, a lysate thereof, or an extract thereof.

In addition, the present invention provides a method of preventing or treating inflammatory diseases, including administering a composition including a *Lactobacillus plantarum* WiKim0175 strain, a culture thereof, a lysate thereof, or an extract thereof as an active ingredient to a subject.

In addition, the present invention provides the use of a composition including, as an active ingredient, a *Lactobacillus plantarum* WiKim0175 strain, a culture thereof, a lysate thereof, or an extract thereof for the manufacture of a pharmaceutical composition for preventing or treating inflammatory diseases.

### [Advantageous Effects]

According to the present invention, *Lactobacillus plantarum* WiKim0175, which is a lactic acid bacterium isolated from kimchi, reduces the expression of inflammation-related and cartilage damage-related genes, thereby inhibiting cartilage matrix degradation. Therefore, *Lactobacillus plantarum* WiKim0175 improves joint and cartilage function and exhibits osteoarthritis-alleviating activity, and thus can be variously utilized for preventing, ameliorating, and treating inflammatory diseases using the lactic acid bacterium as an active ingredient.

### [Brief description of Drawings]

FIG. 1 is graphs showing numerical values converted from the duration (sec, (a)) and the stimulus intensity (PW, (b)) corresponding to the paw withdrawal threshold in response to mechanical stimulus in an osteoarthritis mouse experimental group;
FIG. 2 is a graph showing measurement results of hind limb weight-bearing values in the osteoarthritis mouse experimental group;
FIG. 3 is photographs (a) showing observation of knee tissue damage in the osteoarthritis mouse experimental group and a graph (b) showing measurement results of knee tissue thickness;
FIG. 4 is graphs showing the expression levels of cartilage damage-related genes in damaged cartilage tissue of the osteoarthritis mouse experimental group; and
FIG. 5 is graphs showing the expression levels of inflammation-related genes in damaged cartilage tissue of the osteoarthritis mouse experimental group.

### [Mode for Invention]

A better understanding of the present invention may be obtained through the following examples. These examples are merely set forth to illustrate the present invention, and are not to be construed as limiting the scope of the present invention.

Through the examples of the present invention, the strain isolated from kimchi was shown to have the nucleic acid sequence of SEQ ID NO: 1 as a result of 16S rDNA sequence analysis for microbial identification and classification.

Accordingly, the microorganism of the present invention having the 16S rDNA sequence of SEQ ID NO: 1 was designated as *Lactobacillus plantarum* WiKim0175, and was deposited with the Korean Culture Center of Microorganisms (KCCM) on March 23, 2022 (Accession No. KCCM13151P).

SEQ ID NO: 1

TCAGGACGAACGCTGGCGGCGTGCCTAATACATGCAAGTCGAACGAACTCTGGTATTGATT
GGTGCTTGCATCATGATTTACATTTGAGTGAGTGGCGAACTGGTGAGTAACACGTGGGAAACCTGC
CCAGAAGCGGGGGATAACACCTGGAAACAGATGCTAATACCGCATAACAACTTGGACCGCATGGTC
CGAGCTTGAAAGATGGCTTCGGCTATCACTTTTGGATGGTCCCGCGGCGTATTAGCTAGATGGTGGG
GTAACGGCTCACCATGGCAATGATACGTAGCCGACCTGAGAGGGTAATCGGCCACATTGGGACTGA
GACACGGCCCAAACTCCTACGGGAGGCAGCAGTAGGGAATCTTCCACAATGGACGAAAGTCTGATG
GAGCAACGCCGCGTGAGTGAAGAAGGGTTTCGGCTCGTAAAACTCTGTTGTTAAAGAAGAACATAT
CTGAGAGTAACTGTTCAGGTATTGACGGTATTTAACCAGAAAGCCACGGCTAACTACGTGCCAGCA
GCCGCGGTAATACGTAGGTGGCAAGCGTTGTCCGGATTTATTGGGCGTAAAGCGAGCGCAGGCGGT
TTTTTAAGTCTGATGTGAAAGCCTTCGGCTCAACCGAAGAAGTGCATCGGAAACTGGGAAACTTGA
GTGCAGAAGAGGACAGTGGAACTCCATGTGTAGCGGTGAAATGCGTAGATATATGGAAGAACACC
AGTGGCGAAGGCGGCTGTCTGGTCTGTAACTGACGCTGAGGCTCGAAAGTATGGGTAGCAAACAGG
ATTAGATACCCTGGTAGTCCATACCGTAAACGATGAATGCTAAGTGTTGGAGGGTTTCCGCCCTTCA
GTGCTGCAGCTAACGCATTAAGCATTCCGCCTGGGGAGTACGGCCGCAAGGCTGAAACTCAAAGGA
ATTGACGGGGGCCCGCACAAGCGGTGGAGCATGTGGTTTAATTCGAAGCTACGCGAAGAACCTTAC
CAGGTCTTGACATACTATGCAAATCTAAGAGATTAGACGTTCCCTTCGGGGACATGGATACAGGTG
GTGCATGGTTGTCGTCAGCTCGTGTCGTGAGATGTTGGGTTAAGTCCCGCAACGAGCGCAACCCTTA
TTATCAGTTGCCAGCATTAAGTTGGGCACTCTGGTGAGACTGCCGGTGACAAACCGGAGGAAGGTG
GGGATGACGTCAAATCATCATGCCCCTTATGACCTGGGCTACACACGTGCTACAATGGATGGTACAA
CGAGTTGCGAACTCGCGAGAGTAAGCTAATCTCTTAAAGCCATTCTCAGTTCGGATTGTAGGCTGCA
ACTCGCCTACATGAAGTCGGAATCGCTAGTAATCGCGGATCAGCATGCCGCGGTGAATACGTTCCC
GGGCCTTGTACACACCGCCCGTCA

*Lactobacillus plantarum* WiKim0175 of the present invention is a Gram-positive bacterium and a facultative anaerobe capable of growing under both aerobic and anaerobic conditions, does not form spores, is non-motile, and has rod-shaped cells.

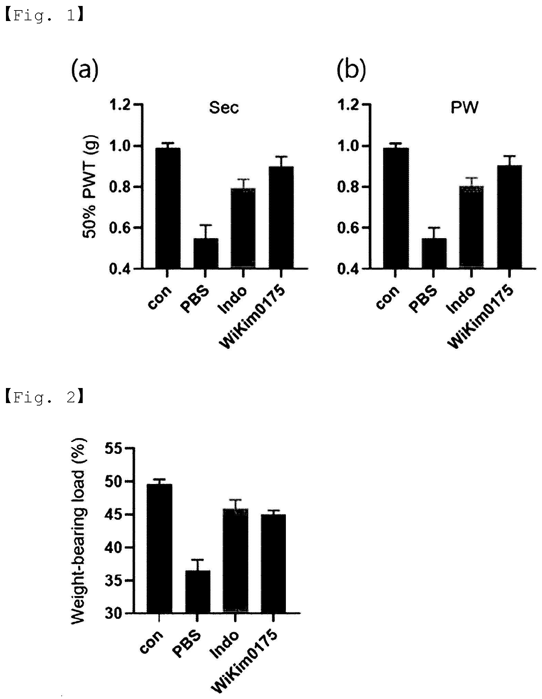

In the following examples, it was confirmed that, in mice with osteoarthritis induced by administration of MIA (monosodium iodoacetate), the group administered *Lactobacillus plantarum* WiKim0175 of the present invention showed a great effect of ameliorating osteoarthritis pain compared to the control group administered PBS (FIGs. 1 and 2). It was also confirmed that administration of *Lactobacillus plantarum* WiKim0175 reduced the expression of cartilage damage-related genes (Adamts4, Adamts5, MMP3, MMP9, and MMP13) and inflammation-related genes (IL-1β, IL-6, TNF-α, COX-2, and iNOS). Therefore, *Lactobacillus plantarum* WiKim0175 of the present invention may be variously utilized for improving joint and cartilage health in humans or animals, preventing or ameliorating arthritic diseases, reducing inflammation, and preventing or ameliorating inflammatory diseases.

The present invention provides a food composition for reducing inflammation or improving joint and cartilage health, including, as an active ingredient, a *Lactobacillus plantarum* WiKim0175 strain, a culture thereof, a lysate thereof, or an extract thereof.

The food composition according to the present invention may reduce inflammation or improve joint and cartilage health by inhibiting the expression of at least one gene selected from the group consisting of Adamts4, Adamts5, MMP3, MMP9, MMP13, IL-1β, IL-6, TNF-α, COX-2, and iNOS.

The food composition may be in the form of a beverage or a bar.

In the present invention, the food composition containing the strain as an active ingredient may include beverages such as fermented milk.

The food composition according to the present invention may be formulated in the same manner as a pharmaceutical composition and used as a functional food, or may be added to various foods. Examples of foods to which the composition of the present invention may be added include beverages, vitamin complexes, and health supplements.

The food composition of the present invention may include components commonly added during food preparation, for example, proteins, carbohydrates, fats, nutrients, seasonings, and flavoring agents. Examples of the carbohydrates include monosaccharides such as glucose and fructose; disaccharides such as maltose, sucrose, and oligosaccharides; and polysaccharides, for example, sugars such as dextrin and cyclodextrin and sugar alcohols such as xylitol, sorbitol, and erythritol. As flavoring agents, natural flavoring agents [thaumatin, stevia extract (e.g., rebaudioside A and glycyrrhizin)] and synthetic flavoring agents (saccharin and aspartame) may be used. For example, when the food composition of the present invention is prepared as a drink formulation or beverage, citric acid, liquid fructose, sugar, glucose, acetic acid, malic acid, fruit juice, and various plant extracts may be additionally included.

The food composition may further include food additives, and suitability as a "food additive" is determined according to the standards and criteria for the relevant item based on the General Provisions and General Test Methods of the Korean Food Additives Code approved by the Ministry of Food and Drug Safety, unless specified otherwise.

Examples of items listed in the Food Additives Code may include chemically synthesized products such as ketones, glycine, potassium citrate, nicotinic acid, and cinnamic acid, natural additives such as persimmon pigment, licorice extract, crystalline cellulose, and guar gum, and mixed preparations such as monosodium L-glutamate preparations, alkaline agents for noodles, preservative preparations, and colorant preparations.

Examples of foods containing the active ingredient of the present invention may include confectionery products such as bread, rice cakes, dried confectionery, candies, chocolates, chewing gum, and jams, ice cream products such as ice cream, frozen desserts, and ice cream powder, dairy products such as milk, low-fat milk, lactose-hydrolyzed milk, processed milk, goat milk, fermented milk, butter oil, concentrated milk, cream, buttermilk, natural cheese, processed cheese, milk powder, and whey products, meat products such as processed meat products, processed egg products, and hamburgers, fish products such as processed fish products including fish cakes, fish ham, fish sausages, and fish bacon, noodle products such as ramen, dried noodles, fresh noodles, fried noodles, pregelatinized dried noodles, retort-processed noodles, frozen noodles, and pasta, beverages such as fruit beverages, vegetable beverages, carbonated beverages, soy milk, lactic acid bacteria beverages such as yogurt, and mixed beverages, seasoning foods such as soy sauce, soybean paste, red pepper paste, black bean paste, cheonggukjang, mixed paste products, vinegar, sauces, tomato ketchup, curry, and dressings, margarine, shortening, and pizza, but are not limited thereto.

In addition, the composition of the present invention may further include various nutrients, vitamins, electrolytes, flavor enhancers, coloring agents, pectic acid and salts thereof, alginic acid and salts thereof, organic acids, protective colloidal thickeners, pH adjusting agents, stabilizers, preservatives, glycerin, alcohol, carbonation agents for use in carbonated beverages, and the like. The composition of the present invention may further include pulp for the preparation of natural fruit juices, fruit juice beverages, and vegetable beverages. These components may be used alone or in combination.

The beverage composition containing the active ingredient of the present invention is not particularly limited with respect to other components, and may further contain various flavoring agents or natural carbohydrates as in typical beverages. Examples of the natural carbohydrates include monosaccharides (e.g., glucose and fructose); disaccharides (e.g., maltose and sucrose); polysaccharides, for example sugars (e.g., dextrin and cyclodextrin) and sugar alcohols such as xylitol, sorbitol, and erythritol. As flavoring agents other than those described above, natural flavoring agents (thaumatin and stevia extracts such as rebaudioside A and glycyrrhizin) and synthetic flavoring agents (saccharin and aspartame) may be advantageously used.

The present invention provides a functional health food composition for reducing inflammation or improving joint and cartilage health, including, as an active ingredient, a *Lactobacillus plantarum* WiKim0175 strain, a culture thereof, a lysate thereof, or an extract thereof.

The functional health food composition according to the present invention may reduce inflammation or improve joint and cartilage health by inhibiting the expression of at least one gene selected from the group consisting of Adamts4, Adamts5, MMP3, MMP9, MMP13, IL-1β, IL-6, TNF-α, COX-2, and iNOS.

The term "functional health food" refers to a food manufactured or processed using raw materials or ingredients with functionality beneficial to the human body, in accordance with the Functional Health Food Act (Article 3, Item 1). The term "functionality" refers to obtaining beneficial effects for health purposes, such as regulating nutrients or exerting physiological actions on the structure and function of the human body (Item 2 of the same Article).

The present invention provides a lactic acid bacteria starter culture for fermentation, including a *Lactobacillus plantarum* WiKim0175 strain or a culture thereof.

The present invention also provides a feed or feed additive composition for reducing inflammation or improving joint and cartilage health, including, as an active ingredient, a *Lactobacillus plantarum* WiKim0175 strain, a culture thereof, a lysate thereof, or an extract thereof.

The feed or feed additive composition according to the present invention may reduce inflammation or improve joint and cartilage health by inhibiting the expression of at least one gene selected from the group consisting of Adamts4, Adamts5, MMP3, MMP9, MMP13, IL-1β, IL-6, TNF-α, COX-2, and iNOS.

When used as a feed additive, the composition may be formulated as a highly concentrated solution of 20 to 90%, or in powder or granule form. The feed additive may further include any one or more selected from among organic acids such as citric acid, fumaric acid, adipic acid, lactic acid, and malic acid, phosphates such as sodium phosphate, potassium phosphate, acidic pyrophosphate, and polyphosphate (condensed phosphate), and natural antioxidants such as polyphenols, catechins, alpha-tocopherol, rosemary extract, vitamin C, green tea extract, licorice extract, chitosan, tannic acid, and phytic acid. When used as feed, the composition may be formulated into a typical feed form and may further include typical feed components.

The feed additive and feed may further include dry ingredients, for example, grains such as ground or crushed wheat, oats, barley, corn, and rice; plant protein feed ingredients such as rapeseed-, soybean-, and sunflower-based feed ingredients; animal protein feed ingredients such as blood meal, meat meal, bone meal, and fish meal; and sugars and dairy products such as various types of milk powder and whey powder. In addition, the composition may further include nutritional supplements, digestion and absorption enhancers, and growth promoters.

The feed additive may be administered to animals alone or in combination with other feed additives in an edible carrier. In addition, the feed additive may be administered as a top dressing, may be directly mixed with animal feed, or may be easily administered to animals in an oral dosage form separate from feed. When the feed additive is administered separately from animal feed, it may be prepared into an immediate-release or sustained-release formulation in combination with a pharmaceutically acceptable edible carrier, as is well known in the art. Such edible carriers may include solid or liquid, for example corn starch, lactose, sucrose, soy flakes, peanut oil, olive oil, sesame oil, and propylene glycol. When a solid carrier is used, the feed additive may be in the form of a tablet, capsule, powder, troche, sugar-coated tablet, or a top dressing in a finely dispersed form. When a liquid carrier is used, the feed additive may be formulated into a soft gelatin capsule, syrup, suspension, emulsion, or solution.

In addition, the feed additive and feed may contain adjuvants such as preservatives, stabilizers, wetting agents, emulsifiers, or solubilizing agents. The feed additive may be added to animal feed by immersion, spraying, or mixing.

The feed or feed additive of the present invention may be applied to diets for a wide range of animals, including mammals, poultry, and fish.

In the present invention, animals include mammals, and the mammals may include pigs, cattle, sheep, goats, laboratory rodents, and companion animals (e.g., dogs and cats), but are not limited thereto.

The present invention provides a pharmaceutical composition for preventing or treating an inflammatory disease, including, as an active ingredient, a *Lactobacillus plantarum* WiKim0175 strain, a culture thereof, a lysate thereof, or an extract thereof.

The inflammatory disease of the present invention may be associated with the expression of at least one inflammation-related gene selected from the group consisting of IL-1β, IL-6, TNF-α, COX-2, and iNOS.

The inflammatory disease of the present invention may include at least one selected from the group consisting of gastritis, stomatitis, atopic dermatitis, acne, psoriasis, sinusitis, rhinitis, conjunctivitis, asthma, dermatitis, inflammatory collagen vascular disease, glomerulonephritis, encephalitis, inflammatory enteritis, chronic obstructive pulmonary disease, sepsis, septic shock, pulmonary fibrosis, undifferentiated spondyloarthropathy, undifferentiated arthropathy, arthritis, osteoarthritis, inflammatory osteolysis, chronic inflammatory diseases caused by viral or bacterial infections, ulcerative colitis, inflammatory bowel disease, rheumatoid arthritis, reactive arthritis, osteoarthritis, scleroderma, osteoporosis, atherosclerosis, myocarditis, endocarditis, pericarditis, cystic fibrosis, Hashimoto's thyroiditis, Graves' disease, leprosy, syphilis, Lyme disease, borreliosis, neuroborreliosis, tuberculosis, sarcoidosis, lupus, chilblain lupus, tuberculous lupus, lupus nephritis, systemic lupus erythematosus, macular degeneration, uveitis, irritable bowel syndrome, Crohn's disease, Sjogren's syndrome, fibromyalgia, chronic fatigue syndrome, chronic fatigue immune dysfunction syndrome, myalgic encephalomyelitis, amyotrophic lateral sclerosis, Parkinson's disease, and multiple sclerosis.

The present invention provides a pharmaceutical composition for preventing or ameliorating an arthritic disease, including, as an active ingredient, a *Lactobacillus plantarum* WiKim0175 strain, a culture thereof, a lysate thereof, or an extract thereof.

The arthritic disease of the present invention may be associated with the expression of at least one gene selected from the group consisting of Adamts4, Adamts5, MMP3, MMP9, MMP13, IL-1β, IL-6, TNF-α, COX-2, and iNOS.

The *Lactobacillus plantarum* WiKim0175 strain included in the composition according to the present invention may be present as viable cells or non-viable cells, and may also be present in a dried or freeze-dried form. In addition, a culture of the *Lactobacillus plantarum* WiKim0175 strain may serve as an active ingredient, and the culture may include a culture broth of viable cells or a cell-free supernatant derived from non-viable cells. The forms of lactic acid bacteria suitable for inclusion in various compositions and methods for formulation are well known to those skilled in the art. For example, *Lactobacillus plantarum* WiKim0175 may be formulated as a culture obtained by cultivation in a known liquid or solid medium, a fermentation product obtained by co-culturing the strain with additional components, an extract obtained by extracting the strain with an organic solvent, or a lysate (or disrupted product) obtained by lysing, disrupting, or homogenizing the cell membrane of the strain, but is not limited thereto.

When the composition according to the present invention is used as a pharmaceutical composition, the pharmaceutical composition of the present invention may be formulated using pharmaceutically suitable and physiologically acceptable adjuvants in addition to the *Lactobacillus plantarum* WiKim0175 strain. Such adjuvants may include excipients, disintegrants, sweeteners, binders, coatings, expanders, lubricants, glidants, or flavoring agents.

The pharmaceutical composition may further include one or more pharmaceutically acceptable carriers in addition to the active ingredient described above for administration purposes, thereby being preferably formulated into a pharmaceutical composition.

For example, for formulation into tablets or capsules, the active ingredient may be combined with orally non-toxic, pharmaceutically acceptable inert carriers such as ethanol, glycerol, or water. If desired or necessary, suitable binders, lubricants, disintegrants, and coloring agents may also be included in the mixture. Suitable binders include, but are not limited to, natural sugars such as starch, gelatin, glucose, or β-lactose, natural and synthetic gums such as corn-based sweeteners, acacia, tragacanth, or sodium oleate, sodium stearate, magnesium stearate, sodium benzoate, sodium acetate, and sodium chloride. Disintegrants include, but are not limited to, starch, methylcellulose, agar, bentonite, and xanthan gum. For liquid formulations, pharmaceutically acceptable carriers may include sterile and physiologically compatible carriers such as saline, sterile water, Ringer's solution, buffered saline, albumin injection solution, dextrose solution, maltodextrin solution, glycerol, ethanol, or combinations of one or more thereof. If necessary, other typical additives such as antioxidants, buffers, and bacteriostatic agents may be added. In addition, diluents, dispersing agents, surfactants, binders, and lubricants may be further added to formulate injectable formulations such as aqueous solutions, suspensions, and emulsions, as well as pills, capsules, granules, or tablets.

Furthermore, the composition is preferably formulated depending on each disease or component using appropriate methods in the art, such as those disclosed in Remington's Pharmaceutical Sciences, Mack Publishing Company, Easton, PA.

### [Examples]

### Example 1: Isolation and culture of Lactobacillus plantarum WiKim0175

The present inventors isolated lactic acid bacteria from kimchi. A kimchi sample was homogenized, and the undiluted kimchi extract was spread onto MRS agar medium (Difco), followed by incubation at 30°C for at least 24 hours. After incubation, single colonies were obtained and subcultured, and final identification was performed through 16S rDNA sequence analysis.

A single colony of *Lactobacillus plantarum* cultured on MRS agar medium (Difco) was further cultured in MRS medium at 30°C for 24 hours. The bacterial cells were then harvested by centrifugation at 8,000 rpm for 5 minutes and washed with PBS to remove residual medium components.

The strain isolated in this example was identified as having a nucleic acid sequence of SEQ ID NO: 1 based on 16S rDNA sequence analysis for microbial identification.

Accordingly, the microorganism of the present invention was designated as *Lactobacillus plantarum* WiKim0175, and was deposited with the Korean Culture Center of Microorganisms on March 23, 2022 (Accession No. KCCM13151P).

### Example 2: Mechanical stimulus-induced withdrawal response and hind limb weight-bearing test

To evaluate the osteoarthritis-ameliorating effect of *Lactobacillus plantarum* WiKim0175 of the present invention, experimental groups were prepared using 8-week-old male C57BL/6N mice according to the following procedure.

### 1)- Normal mice

1-1) After anesthesia by inhalation, the hair around the left knee of the mouse was removed, and 10 µl of PBS was injected into the knee joint cavity (PBS group).

### 2)- Osteoarthritis-induced mice

After anesthesia by inhalation, the hair around the left knee of the mouse was removed and 1 mg/10 µl of MIA was injected once into the knee joint cavity to induce osteoarthritis, after which indomethacin or WiKim0175 was administered.

2-1) After intra-articular injection of MIA, which is an osteoarthritis-inducing substance, indomethacin was orally administered at 2 mg/kg five times per week for a total of 3 weeks (Indo group).

2-2) WiKim0175 was orally administered at 2x10⁹ CFU five times per week for a total of 5 weeks, starting 2 weeks before the induction of osteoarthritis and continuing for 3 weeks after intra-articular injection of MIA (WiKim0175 group).

To evaluate the osteoarthritis pain-ameliorating effect in the osteoarthritis mouse groups prepared by the above procedure, the paw withdrawal threshold (PWT, in this experiment expressed as 50% PWT, a measure of the pain threshold) in response to mechanical stimulus was measured using a mechanical hypersensitivity (allodynia) test. The measurement was performed as follows.

The plantar surface of the paw of each mouse in each group was stimulated using a blunt-tipped probe, and the duration (sec) for which pain could be tolerated and the level of tolerable pain intensity (PW) as the stimulus intensity gradually increased were measured. As pain was induced, the duration (sec) for tolerating the stimulus became shorter, and the magnitude of the tolerable stimulus value (PW) became lower. The measured values are shown in FIG. 1.

FIG. 1(a) shows values obtained by converting the duration (sec) for tolerating pain induced by mechanical stimulus into a threshold value, and FIG. 1(b) shows values obtained by converting the tolerable pain intensity (PW) of mechanical stimulus into a threshold value.

In the WiKim0175 group, compared to the PBS group, the paw withdrawal threshold (PWT) increased with duration in FIG. 1(a) and with stimulus intensity in FIG. 1(b). In addition, compared to the Indo group as the positive control group, the PWT in response to stimulus intensity was comparable or high. These results confirm the osteoarthritis pain-ameliorating effect of WiKim0175.

Furthermore, the amelioration of osteoarthritis pain was also confirmed by measuring hind limb weight-bearing values using a weight-bearing load test. In the WiKim0175 group, the weight-bearing value increased compared to the PBS group, and also increased compared to the Indo group as the positive control group. These results likewise confirm the osteoarthritis pain-ameliorating effect of WiKim0175 (FIG. 2).

### Example 3: Observation of knee tissue damage

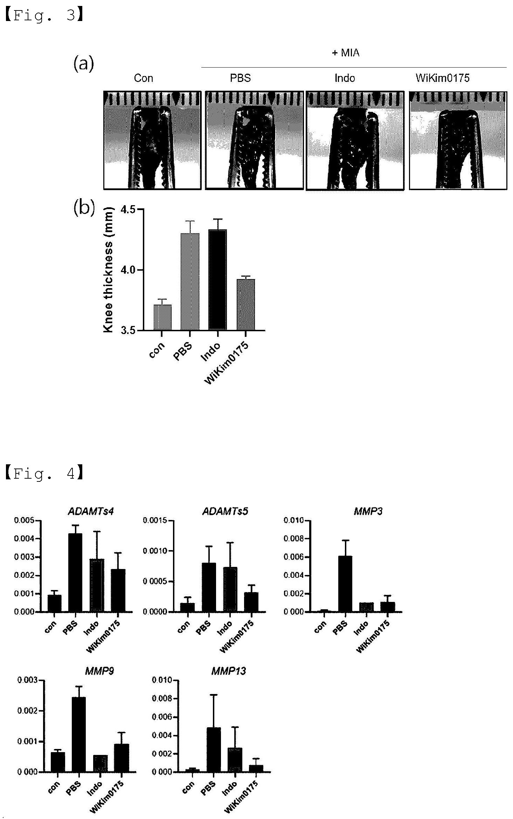

Knee tissue damage in the osteoarthritis mouse groups prepared in Example 2 was observed (FIG. 3(a)). In cases of osteoarthritis, damage occurs in the knee tissue, resulting in an increase in knee thickness.

In the WiKim0175 group, the knee tissue thickness decreased compared to the PBS group, confirming amelioration of joint damage (FIG. 3(b)).

### Example 4: Effects of Lactobacillus plantarum WiKim0175 on expression levels of cartilage damage-related and inflammation-related genes

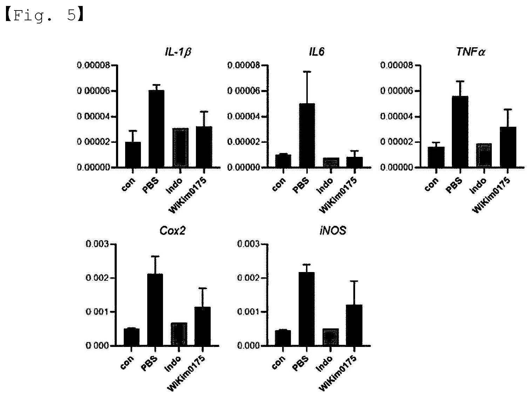

Cartilage tissue damaged by MIA injection was collected from the osteoarthritis mice of the above examples, and the expression levels of cartilage damage-related genes (Adamts4, Adamts5, MMP3, MMP9, and MMP13) and inflammation-related genes (IL-1β, IL-6, TNF-α, COX-2, and iNOS) were analyzed. In the cartilage tissue of the WiKim0175 group, reduced expression of cartilage damage-related and inflammation-related genes was observed. The WiKim0175 group showed a significant inhibitory effect on the expression of the cartilage damage-related and inflammation-related genes compared to the PBS group (FIGs. 4 and 5).
Depositing institution: Korean Culture Center of Microorganisms (KCCM)
Accession No.: KCCM13151P
Deposit date: 2022-03-23

(Translation)

Budapest Treaty on the International Recognition of the Deposit of

Microorganisms for the Purposes of Patent Procedure

INTERNATIONAL FORM

RECEIPT FOR ORIGINAL DEPOSIT
issued pursuant to Rule 7.1

Recipient: Korea Food Research Institute
245beon-gil Nongsaengmyeong-ro, Iseo-myeon, Wanju-gun, Jeollabuk-do, Republic
of Korea (55365)

I. IDENTIFICATION OF THE MICROORGANISM
Identification reference given by the
DEPOSITOR:
Lactobacillus plantarum WiKim0175 | Accession number given by the
INTERNATIONAL DEPOSITARY AUTHORITY:
KCCM13151P
II. SCIENTIFIC DESCRIPTION AND PROPOSED TAXONOMIC
DESIGNATION
The microorganism identified under I above was accompanied by:
[ ] a scientific description
[ ] a proposed taxonomic designation
III. RECEIPT AND ACCEPTANCE
This International Depositary Authority accepts the microorganism identified
under I above, which was received thereby on March 23, 2022.
IV. INTERNATIONAL DEPOSITARY AUTHORITY
Name: Korean Culture Center of
Microorganisms

Address: Korean Culture Center of
Microorganisms (KCCM)
45 Yurim Building, Hongjenae 2-ga-gil,
Seodaemun-gu, Seoul, Republic of Korea
(03641) | Representative's signature:

March 23, 2022

The above translation is identical to the
original document |

## Claims

1. *Lactobacillus plantarum* WiKim0175 deposited under Accession No. KCCM13151P.

2. A food composition for reducing inflammation or improving joint and cartilage health, comprising, as an active ingredient, *Lactobacillus plantarum* WiKim0175 (Accession No. KCCM13151P), a culture thereof, a lysate thereof, or an extract thereof.

3. The food composition according to claim 2, wherein the food composition reduces inflammation or improves joint and cartilage health by inhibiting expression of at least one gene selected from the group consisting of Adamts4, Adamts5, MMP3, MMP9, MMP13, IL-1β, IL-6, TNF-α, COX-2, and iNOS.

4. A functional health food composition for reducing inflammation or improving joint and cartilage health, comprising, as an active ingredient, *Lactobacillus plantarum* WiKim0175 (Accession No. KCCM13151P), a culture thereof, a lysate thereof, or an extract thereof.

5. The functional health food composition according to claim 4, wherein the functional health food composition reduces inflammation or improves joint and cartilage health by inhibiting expression of at least one gene selected from the group consisting of Adamts4, Adamts5, MMP3, MMP9, MMP13, IL-1β, IL-6, TNF-α, COX-2, and iNOS.

6. A lactic acid bacteria starter culture for fermentation, comprising *Lactobacillus plantarum* WiKim0175 (Accession No. KCCM13151P) or a culture thereof.

7. A feed or feed additive composition comprising, as an active ingredient, *Lactobacillus plantarum* WiKim0175 (Accession No. KCCM13151P), a culture thereof, a lysate thereof, or an extract thereof.

8. A pharmaceutical composition for preventing or treating an inflammatory disease, comprising, as an active ingredient, *Lactobacillus plantarum* WiKim0175 (Accession No. KCCM13151P), a culture thereof, a lysate thereof, or an extract thereof.

9. The pharmaceutical composition according to claim 8, wherein the inflammatory disease is associated with expression of at least one inflammation-related gene selected from the group consisting of IL-1β, IL-6, TNF-α, COX-2, and iNOS.

10. The pharmaceutical composition according to claim 8, wherein the inflammatory disease comprises at least one selected from the group consisting of gastritis, stomatitis, atopic dermatitis, acne, psoriasis, sinusitis, rhinitis, conjunctivitis, asthma, dermatitis, inflammatory collagen vascular disease, glomerulonephritis, encephalitis, inflammatory enteritis, chronic obstructive pulmonary disease, sepsis, septic shock, pulmonary fibrosis, undifferentiated spondyloarthropathy, undifferentiated arthropathy, arthritis, osteoarthritis, inflammatory osteolysis, chronic inflammatory diseases caused by viral or bacterial infections, ulcerative colitis, inflammatory bowel disease, rheumatoid arthritis, reactive arthritis, scleroderma, osteoporosis, atherosclerosis, myocarditis, endocarditis, pericarditis, cystic fibrosis, Hashimoto's thyroiditis, Graves' disease, leprosy, syphilis, Lyme disease, borreliosis, neuroborreliosis, tuberculosis, sarcoidosis, lupus, chilblain lupus, tuberculous lupus, lupus nephritis, systemic lupus erythematosus, macular degeneration, uveitis, irritable bowel syndrome, Crohn's disease, Sjogren's syndrome, fibromyalgia, chronic fatigue syndrome, chronic fatigue immune dysfunction syndrome, myalgic encephalomyelitis, amyotrophic lateral sclerosis, Parkinson's disease, and multiple sclerosis.

11. The pharmaceutical composition according to claim 8, wherein the inflammatory disease is an arthritic disease.

12. The pharmaceutical composition according to claim 11, wherein the arthritic disease is associated with expression of at least one gene selected from the group consisting of Adamts4, Adamts5, MMP3, MMP9, MMP13, IL-1β, IL-6, TNF-α, COX-2, and iNOS.

13. The pharmaceutical composition according to claim 11, wherein the arthritic disease comprises at least one selected from the group consisting of undifferentiated spondyloarthropathy, undifferentiated arthropathy, arthritis, osteoarthritis, inflammatory osteolysis, rheumatoid arthritis, and reactive arthritis.
